## Europäisches Patentamt
## European Patent Office
## Office européen des brevets

(19)

(11) Veröffentlichungsnummer: **0 282 633 B1**

# EUROPÄISCHE PATENTSCHRIFT

(12)

(45) Veröffentlichungstag der Patentschrift: **08.05.91**

(51) Int. Cl.⁵: **A61K 6/08**

(21) Anmeldenummer: **87114635.3**

(22) Anmeldetag: **07.10.87**

(54) Kunststoff-Zahnersatzteil.

(30) Priorität: **17.03.87 DE 3708618**

(43) Veröffentlichungstag der Anmeldung:
**21.09.88 Patentblatt 88/38**

(45) Bekanntmachung des Hinweises auf die
Patenterteilung:
**08.05.91 Patentblatt 91/19**

(84) Benannte Vertragsstaaten:
**AT BE CH DE ES FR GB GR IT LI LU NL SE**

(56) Entgegenhaltungen:
**EP-A- 0 166 009**
**WO-A-81/02254**
**DE-A- 2 405 578**
**DE-A- 2 914 537**

(73) Patentinhaber: **Heraeus Kulzer GmbH**
**Heraeusstr. 12 - 14**
**W-6450 Hanau(DE)**

(72) Erfinder: **Schaefer, Roland, Dr.**
**Merianweg 5**
**W-6382 Friedrichsdorf(DE)**

(74) Vertreter: **Grimm, Ekkehard**
**Heraeus Holding GmbH Heraeusstrasse 12 -
14**
**W-6450 Hanau/Main(DE)**

## Beschreibung

Die Erfindung betrifft ein Kunststoff-Zahnersatzteil mit einem Mantel aus einem 10 - 90 Gewichts-% hochdisperses Siliciumdioxid mit einer Teilchengröße von 0,01 - 0,4 Mikrometer enthaltenden Kunststoff aus einem Polymer auf Acrylat- und/oder Methacrylat-Basis.

Aus der deutschen Offenlegungsschrift 24 05 578 sind Zahnersatzteile - Kronen, Brücken, künstliche Zähne -, deren äußere Schicht aus einem amorphe Kieselsäure - vorteilhafterweise 30 - 80 Gewichts-% - mit einer maximalen Teilchengröße von 0,07 Mikrometer enthaltenden Kunststoff besteht, bekannt. Diese Schicht besitzt eine hohe Abrasionsfestigkeit und kann auf Hochglanz poliert werden. Sie wird aus polymerisierbaren Mischungen der amorphen Kieselsäure, besonders mit Estern der Methacrylsäure, zum Beispiel Bis-[4-(2-hydroxy-3-methacryloyloxypropoxy)]-dimethylmethan, das sogenannte Bis-GMA (siehe US-Patentschrift 3 066 112), und Triäthylenglykoldimethacrylat, hergestellt. Amorphe Kieselsäure dieser Art oder einen anderen anorganischen Füllstoff entsprechender Teilchengröße enthaltende Dentalmaterialien auf Kunststoff-Basis werden auch als Composites mit mikrofeinem Füllstoff oder mit Mikrofüller (microfiller) bezeichnet.

Aus der britischen Patentschrift 1 488 403 sind ebenfalls Zahnersatzteile, deren äußere Schicht aus einem einen mikrofeinen anorganischen Füllstoff, besonders aus Siliciumdioxid und/oder Aluminiumoxid, enthaltenden Kunststoff besteht, bekannt. Das Material zur Herstellung der Schicht enthält als polymerisierbares Monomer und/oder Polymer Polystyrol, Polyamide, Epoxy-Verbindungen, Polyurethane, monomere und polymere Acrylate und Methacrylate oder Mischungen daraus und 10 - 90 Gewichts-% des mikrofeinen Füllstoffes mit einer Teilchengröße von 0,01 - 04, Mikrometer.

Mit konventionellem oder mikrofein gefülltem Kronen- und Brückenkunststoff ummantelte Kunststoffbrücken für die semipermanente Rekonstruktion werden in Acta Paradontologica Vol. 11, Nr. 3, 77 - 87, 1982, beschrieben. Diese Brücken sind mit einem gespritzten Polyester-Kern verstärkt und besitzen dadurch eine sehr gute Bruchfestigkeit. Nach Ansicht der Verfasser ist dennoch die Entwicklung von neuen Materialien, die wesentlich verschleißfester und formstabiler sind und zudem graziler ausgearbeitet werden können, erforderlich.

Sowohl konventionelle als auch mikrofeine anorganische Füllstoffe enthaltende Dentalmaterialien - dafür hat sich die Bezeichnung Hybrid-Composites eingebürgert - werden in der internationalen Patentanmeldung WO 81/02254 beschrieben. Sie enthalten Füllstoff-Gemische aus hydrophobem Siliciumdioxid mit einem Durchmesser von 0,01 - 0,04 Mikrometer und Glas, zum Beispiel röntgenopakes Barium- oder Strontiumhaltiges Glas, mit einem Durchmesser von 2 - 30 Mikrometer. Als polymerisierbare Monomere dienen Bis-GMA oder äthoxyliertes Bisphenol A-dimethacrylat und Triäthylenglykoldimethacrylat. Das Material wird als Zahnfüllungsmaterial und zum Verblenden von zum Beispiel gegossenen Gold-Kronen verwendet.

Ein Verfahren zur einfachen und raschen Herstellung von Kunststoffkronen oder von mit Kunststoff überzogenen Metallkronen wird in der deutschen Offenlegungsschrift 29 14 537 beschrieben. Dazu wird ein photopolymerisierbares Material benutzt, das Acrylate und/oder Methacrylate und gegebenenfalls einen anorganischen Füllstoff, vorzugsweise Quarz, und feines Kieselsäureanhydrid als Eindicker für die Monomeren und als Ausfällungsinhibitor für den Füllstoff enthält. Die Kronen werden durch ein- bis dreimaliges Aufbringen des photopolymerisierbaren Materials auf die Gipsform oder das Metallgerüst und kurzes Belichten, um es auszuhärten, hergestellt. Es werden sowohl einheitlich als auch unterschiedlich aufgebaute Kronen beschrieben. Letztere bestehen aus Kunststoff ohne anorganischen Füllstoff und einer dünnen Beschichtung aus Kunststoff mit anorganischem Füllstoff.

Ein photopolymerisierbares Kronen- und Brückenmaterial, das als Füllstoff vorzugsweise ein Gemisch aus sehr feinem Siliciumdioxid (hochdisperses Siliciumdioxid) und Glas enthält, ist aus der europäischen Patentanmeldung 166 009 bekannt. Als Monomere werden Reaktionsprodukte von Polyisocyanaten mit Bisphenol-diglycidylacrylaten und -methacrylaten und daneben andere Urethangruppen-haltige oder Urethangruppen-freie Aryl- und Methacrylsäureester mehrwertiger Alkohole eingesetzt.

Künstliche Zahnteile aus einem Kunststoff-Kern, einem ihn umgebenden dünnwandigen metallischen Gerüst und einem vorzugsweise aus dem gleichen Kunststoff (Acrylharz) wie der Kern bestehenden Mantel sind aus der deutschen Offenlegungsschrift 34 13 398 bekannt.

Es ist die Aufgabe der Erfindung, ein Zahnersatzteil mit einem Mantel aus einem als anorganischen Füllstoff hochdisperses Siliciumdioxid enthaltenden Kunststoff aus einem Polymer auf Acrylat- und/oder Methacrylat-Basis zu finden, dessen Kern eine höhere mechanische Festigkeit als der Mantel besitzen und ebenfalls aus mit anorganischem Füllstoff gefülltem Kunststoff bestehen soll.

Das die Lösung der Aufgabe darstellende Zahnersatzteil ist dadurch gekennzeichnet, daß der Mantel einen Kern aus einem als anorganischen Füllstoff 30 - 90 Gewichts-% einer Mischung aus 60 - 100 Gewichts-% eines anorganischen Füllstoff-Materials mit einer mittleren Teilchengröße von 0,7 - 5 Mikrometer und 0 - 40 Gewichts-% hochdispersem Siliciumdioxid mit einer Teilchengröße von 0,01 - 0,4 Mikrometer enthaltenden Kunststoff aus einem Polymer auf Acrylat-und/oder Methacrylat-Basis umgibt.

Vorzugsweise enthält der Kern 60 - 85 Gewichts-% des anorganischen Füllstoffs und besteht dieser aus einer Mischung aus 80 - 90 Gewichts-% anorganischem Füllstoff-Material und 10 - 20 Gewichts-% hochdispersem Siliciumdioxid.

Der Mantel enthält vorzugsweise 30 - 70 Gewichts-% hochdisperses Siliciumdioxid.

Hochdisperses Siliciumdioxid mit einer mittleren Teilchengröße von 0,01 - 0,04 Mikrometer hat sich besonders bewährt.

Als anorganisches Füllstoff-Material eignet sich jeder anorganische Füllstoff, der in Kunststoff-Dentalmaterialien verwendet wird. Besonders bewährt haben sich Siliciumdioxid, Lithiumaluminiumsilicat-Glas und/oder Strontiumaluminiumsilicat-Glas.

Es hat sich als vorteilhaft erwiesen, wenn das anorganische Füllstoff-Material und das hochdisperse Siliciumdioxid - sowohl das im Mantel als auch das gegebenenfalls im Kern enthaltene - silanisiert sind, zum Beispiel mit 3-Methacryloyloxypropyl-trimethoxysilan.

Der Kunststoff von Kern und Mantel besteht vorzugsweise aus polymeren Estern der Acrylsäure und/oder Methacrylsäure mit zwei- und/oder mehrwertigen Alkoholen. Besonders geeignet sind Polymere aus Bis-GMA, äthoxyliertem Bisphenol A-diacrylat, äthoxyliertem Bisphenol A-dimethacrylat, Triäthylenglykoldimethacrylat, Dodecandioldimethacrylat, Urethandimethacrylat aus 2-Hydroxyäthylmethacrylat und 2,2,4-Trimethylhexamethylendiisocyanat, Bis-(acryloyloxymethyl)-tricyclo$[5.2.1.0^{2,6}]$decan und/oder Bis-(methacryloyloxymethyl)-tricyclo$[5.2.1.0^{2,6}]$decan. (Zur Verwendung der beiden zuletzt genannten Verbindungen in Dentalmassen siehe deutsche Patentschrift 28 16 823.)

Für den Kern haben sich Polymere aus zu 20 - 85 Gewichts-% aus Bis-GMA oder äthoxyliertem Bisphenol A-dimethacrylat bestehenden Estern der Acrylsäure und/oder Methacrylsäure mit zwei- und/oder mehrwertigen Alkoholen besonders bewährt.

Der Kunststoff des Mantels enthält vorzugsweise ein Polymer aus Bis-(methacryloyloxymethyl)-tricyclo-$[5.2.1.0^{2,6}]$decan und/oder dem Urethandimethacrylat aus 2-Hydroxyäthylmethacrylat und 2,2,4-Trimethylhexamethylendiisocyanat, gegebenenfalls zusammen mit einem Polymer ein Dodecandioldimethacrylat.

Daneben haben sich Polymere aus zu 30 - 80 Gewichts-% aus dem Urethandimethacrylat aus 2-Hydroxyäthylmethacrylat und 2,2,4-Trimethylhexamethylendiisocyanat bestehenden Estern der Acrylsäure und/oder Methacrylsäure mit zwei- und/oder mehrwertigen Alkoholen und aus zu 20 - 70 Gewichts-% aus dem Urethandimethacrylat aus 2-Hydroxyäthylmethacrylat und 2,2,4-Trimethylhexamethylendiisocyanat und zu 20 - 40 Gewichts-% aus Bis-(methacryloyloxymethyl)-tricyclo$[5.2.1.0^{2,6}]$decan bestehenden Estern der Acrylsäure und/oder Methacrylsäure mit zwei- und/oder mehrwertigen Alkoholen besonders bewährt.

Das erfindungsgemäße Zahnersatzteil läßt sich auf Hochglanz polieren und besitzt eine hohe Abrasionsfestigkeit, Biegefestigkeit (Kern: etwa 120 - 200 MPa), Bruchfestigkeit und Formstabilität, so daß sein Einsatz sowohl für die provisorische und semipermanente als auch für permanente Versorgung mit Kronen und Brücken möglich ist. Ebenso gut geeignet ist es zum Beispiel auch als Inlay.

Die sehr guten mechanischen Eigenschaften erlauben unter anderem dünnwandige Formen - ein Vorteil, wenn für die Versorgung mit Kronen wenig Platz zur Verfügung steht - und grazile Ausführungen, wie beispielsweise der interdentalen Verbindungszonen zwischen Krone und Brücke bei Brücken mit Vollzahn-Brückengliedern.

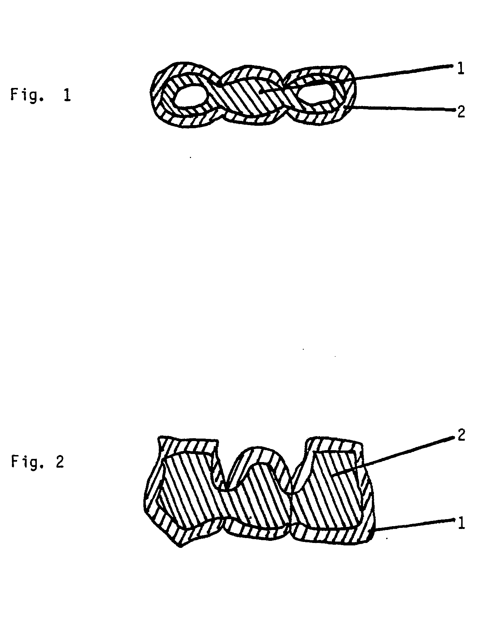

Eine dreigliedrige Brücke mit einem solchen Vollzahn-Brückenglied wird als Beispiel für ein Zahnersatzteil gemäß der Erfindung in den Figuren 1 und 2 dargestellt.

Die Figur 1 zeigt die aus dem Kern 1 und dem Mantel 2 bestehende Brücke in der Draufsicht, die Figur 2 die Brücke im Schnitt.

Das Zahnersatzteil wird aus monomere Ester der Acrylsäure und/oder Methacrylsäure mit zwei- und/oder mehrwertigen Alkoholen, besonders Bis-GMA, äthoxyliertes Bisphenol A-diacrylat, äthoxyliertes Bisphenol A-dimethacrylat, Triäthylenglykoldimethacrylat, Dodecandioldimethacrylat, Urethandimethacrylat aus 2-Hydroxyäthylmethacrylat und 2,2,4-Trimethylhexamethylendiisocyanat, Bis-(acryloyloxymethyl)-tricyclo$[5.2.1.0^{2,6}]$decan und/oder Bis-(methacryloyloxymethyl)-tricyclo$[5.2.1.0^{2,6}]$decan, die anorganischen Füllstoffe und Katalysatoren, die die Photopolymerisation bewirken, enthaltenden Mischung - im folgenden als Kernmaterial und Mantelmaterial bezeichnet - hergestellt.

Als Photopolymerisationskatalysatoren haben sich die aus der GB-PS 1 408 265 bekannten Gemische aus Ketonen und Aminen, besonders aus Campherchinon und Aminen, oder Gemische aus Campherchinon, Benzilacetalen und Aminen, zum Beispiel 4-Dimethylaminobenzoesäureäthylester, bewährt. Bei Bestrahlung mit Licht einer Wellenlänge von 320 - 500 Nanometer lösen sie die Polymerisation der Monomeren aus.

Vorzugsweise enthält sowohl das Kernmaterials als auch das Mantelmaterial jeweils 0,1 - 0,5 Gewichts-% des Campherchinons, des Amins und gegebenenfalls des Benzilacetals.

Zur näheren Erläuterung werden in den folgenden Beispielen bevorzugte Zusammensetzungen des Kernmaterials und des Mantelmaterials und die Herstellung eines Zahnersatzteiles gemäß der Erfindung unter Anwendung des Lichtgerätes gerätes Dentacolor XS der Firma Kulzer für die Photopolymerisation von Kern- und Mantelmaterial beschrieben. Das Mantelmaterial enthält einen Teil des hochdispersen Siliciumdioxids in Form eines damit gefüllten Splitterpolymerisats aus 55 Gewichts-% hochdispersem Siliciumdioxid, mittlere Teilchengröße 0,04 Mikrometer, silanisiert mit 3-Methacryloyloxy-propyl-trimethoxysilan, und 45 Gewichts-% Dodecandioldimethacrylat.

## Beispiel 1

### Kernmaterial

| | |
|---|---|
| 4,5 Gewichts-% | Bis-GMA |
| 10,5 Gewichts-% | Triäthylenglykoldimethacrylat |
| 72,6 Gewichts-% | Lithiumaluminiumsilicat-Glas, mittlere Teilchengröße 5 Mikrometer |
| 12,0 Gewichts-% | hochdisperses Siliciumdioxid, mittlere Teilchengröße 0,04 Mikrometer |
| 0,1 Gewichts-% | Campherchinon |
| 0,1 Gewichts-% | Benzildimethylacetal |
| 0,2 Gewichts-% | p-Dimethylaminobenzoesäureäthylester |

## Beispiel 2

### Kernmaterial

| | |
|---|---|
| 12,4 Gewichts-% | äthoxyliertes Bisphenol A-diacrylat |
| 2,8 Gewichts-% | Trimethylolpropantriacrylat |

| | |
|---|---|
| 71,2 Gewichts-% | Lithiumaluminiumsilicat-Glas, mittlere Teilchengröße 5 Mikrometer |
| 13,2 Gewichts-% | hochdisperses Siliciumdioxid, mittlere Teilchengröße 0,04 Mikrometer, silanisiert mit 3-Methacryloyloxypropyl-trimethoxysilan |
| 0,1 Gewichts-% | Campherchinon |
| 0,1 Gewichts-% | Benzildimethylacetal |
| 0,2 Gewichts-% | p-Dimethylaminobenzoesäureäthylester |

## Beispiel 3

Kernmaterial

| | |
|---|---|
| 12,1 Gewichts-% | äthoxyliertes Bisphenol A-dimethacrylat |
| 3,0 Gewichts-% | Hexamethylendioldimethacrylat |
| 79,8 Gewichts-% | Lithiumaluminiumsilicat-Glas, mittlere Teilchengröße 5 Mikrometer |
| 4,7 Gewichts-% | hochdisperses Siliciumdioxid, mittlere Teilchengröße 0,04 Mikrometer, silanisiert mit 3-Methacryloyloxypropyl-trimethoxysilan |
| 0,1 Gewichts-% | Campherchinon |
| 0,1 Gewichts-% | Benzildimethylacetal |
| 0,2 Gewichts-% | p-Dimethylaminobenzoesäureäthylester |

Beispiel 4

Mantelmaterial

11,3 Gewichts-% Urethandimethacrylat aus 2 mol
2-Hydroxyäthylmethacrylat und 1 mol
2,2,4-Trimethylhexamethylendiisocyanat (Plex 6661 der
Firma Röhm, Darmstadt)

7,6 Gewichts-% Bis-GMA

3,4 Gewichts-% Triäthylenglykoldimethacrylat

27,0 Gewichts-% hochdisperses Siliciumdioxid, mittlere Teilchengröße
0,04 Mikrometer, silanisiert mit
3-Methacryloyloxypropyl-trimethoxysilan

50,0 Gewichts-% Splitterpolymerisat, mittlere Teilchengröße 40
Mikrometer

0,1 Gewichts-% Campherchinon

0,1 Gewichts-% Benzildimethylacetal

0,2 Gewichts-% p-Dimethylaminobenzoesäureäthylester

0,3 Gewichts-% Pigmente

Beispiel 5

Mantelmaterial

19,3 Gewichts-%   Bis-(acryloyloxymethyl)-tricyclo$[5.2.1.0^{2,6}]$decan

28,1 Gewichts-%   hochdisperses Siliciumdioxid, mittlere Teilchengröße
0,04 Mikrometer, silanisiert mit
3-Methacryloyloxypropyl-trimethoxysilan

52,0 Gewichts-%   Splitterpolymerisat, mittlere Teilchengröße 40
Mikrometer

0,1 Gewichts-%   Campherchinon

0,1 Gewichts-%   Benzildimethylacetal

0,2 Gewichts-%   p-Dimethylaminobenzoesäureäthylester

0,2 Gewichts-%   Pigmente

Beispiel 6

Mantelmaterial

19,3 Gewichts-%   Bis-(methacryloyloxymethyl)-tricyclo$[5.2.1.0^{2,6}]$decan

28,1 Gewichts-%   hochdisperses Siliciumdioxid, mittlere Teilchengröße
0,04 Mikrometer, silanisiert mit
3-Methacryloyloxypropyl-trimethoxysilan

52,0 Gewichts-%   Splitterpolymerisat, mittlere Teilchengröße 40
Mikrometer

0,1 Gewichts-%   Campherchinon

0,1 Gewichts-%   Benzildimethylacetal

0,2 Gewichts-%   p-Dimethylaminobenzoesäureäthylester

0,2 Gewichts-%   Pigmente

## Beispiel 7

Kern/Mantel-Kronen

1.      Isolierung der Modellstümpfe, z.B. mit Alginat oder Silikonisolierung.

2.      Modellation des Kernmaterials über einem oder mehreren Modellstümpfen, Schichtdicke 0,3 - 0,6 mm.

3.      Aushärtung bzw. Zwischenpolymerisation des Kernmaterials zu einer
tragfähigen Schicht durch 90 Sekunden langes Bestrahlen mit dem
Lichtgerät.

4.      Vollständiger Aufbau der Krone mit dem Mantelmaterial in einer
Schichtdicke von 0,8 - 1,2 mm über dem zwischenpolymerisierten Kernmaterial.

5.      Endpolymerisation durch 180 Sekunden langes Bestrahlen mit dem
Lichtgerät.

6.      Bearbeitung und Hochglanzpolitur.

Zur Bestimmung der Druckfestigkeit von Kern und Mantel des erfindungsgemäßen Zahnersatzteiles werden Proben der in den Beispielen 1 - 6 beschriebenen Kern- und Mantelmaterialien in Glasröhrchen (Innendurchmesser 4 mm, Höhe 8 mm) gegeben und 360 Sekunden lang mit dem Lichtgerät Dentacolor XS bestrahlt. Die so erhaltenen polymeren Prüfkörper werden den Glasröhrchen entnommen und 24 Stunden lang in 37°C warmem Wasser gelagert. Dann wird ihre Druckfestigkeit gemäß der australischen Norm für Zahnfüllungsmaterialien AS 1278 - 1973 gemessen.

Weiter werden aus Proben der in den Beispielen 1- 6 beschriebenen Kern-und Mantelmaterialien polymere Prüfkörper in der Größe 25 x 2 x 2 mm hergestellt. Biegefestigkeit und Biegemodul der Prüfkörper werden nach DIN 13 922 ermittelt.

In der Tabelle werden die erhaltenen Werte für Druckfestigkeit, Biegefestigkeit und Biegemodul angegeben.

EP 0 282 633 B1

Tabelle

| a) Kernmaterial<br>b) Mantelmaterial | Druckfestigkeit<br>[MPa] | Biegefestigkeit<br>[MPa] | Biegemodul<br>[MPa] |
|---|---|---|---|
| **a)** | | | |
| Beispiel 1 | 400 | 180 | 19000 |
| Beispiel 2 | 380 | 160 | 18050 |
| Beispiel 3 | 420 | 183 | 17800 |
| **b)** | | | |
| Beispiel 4 | 400 | 65 | 3500 |
| Beispiel 5 | 420 | 80 | 3600 |
| Beispiel 6 | 400 | 90 | 3900 |

## Ansprüche

1. Kunststoff-Zahnersatzteil mit einem Mantel aus einem 10 - 90 Gewichts-% hochdisperses Siliciumdioxid mit einer Teilchengröße von 0,01 - 0,4 Mikrometer enthaltenden Kunststoff aus einem Polymer auf Acrylat- und/oder Methacrylat-Basis, dadurch gekennzeichnet, daß der Mantel einen Kern aus einem als anorganischen Füllstoff 30 - 90 Gewichts-% einer Mischung aus 60 - 100 Gewichts-% eines anorganischen Füllstoff-Materials mit einer mittleren Teilchengröße von 0,7 - 5 Mikrometer und 0 - 40 Gewichts-% hochdispersem Siliciumdioxid mit einer Teilchengröße von 0,01 - 0,4 Mikrometer enthaltenden Kunststoff aus einem Polymer auf Acrylat- und/oder Methacrylat-Basis umgibt.

2. Zahnersatzteil nach Anspruch 1, dadurch gekennzeichnet, daß der Kern 60 - 85 Gewichts-% des anorganischen Füllstoffs enthält.

3. Zahnersatzteil nach Anspruch 1 oder 2, dadurch gekennzeichnet, daß der anorganische Füllstoff aus einer Mischung aus 80 - 90 Gewichts-% anorganischem Füllstoff-Material und 10 - 20 Gewichts-% hochdispersem Siliciumdioxid besteht.

4. Zahnersatzteil nach einem der Ansprüche 1 - 3, dadurch gekennzeichnet, daß der Mantel 30 - 70 Gewichts-% hochdisperses Siliciumdioxid enthält.

5. Zahnersatzteil nach einem der Ansprüche 1 - 4, dadurch gekennzeichnet, daß das hochdisperse

9

Siliciumdioxid eine mittlere Teilchengröße von 0,01 - 0,04 Mikrometer besitzt.

6. Zahnersatzteil nach einem der Ansprüche 1 - 5, dadurch gekennzeichnet, daß das anorganische Füllstoff-Material aus Siliciumdioxid, Lithiumaluminiumsilicat-Glas und/oder Strontiumaluminiumsilicat-Glas besteht.

7. Zahnersatzteil nach einem der Ansprüche 1 - 6, dadurch gekennzeichnet, daß das anorganische Füllstoff-Material und das hochdisperse Siliciumdioxid silanisiert sind.

8. Zahnersatzteil nach einem der Ansprüche 1 - 7, dadurch gekennzeichnet, daß der Kunststoff von Kern und Mantel aus polymeren Estern der Acrylsäure und/oder Methacrylsäure mit zwei- und/oder mehrwertigen Alkoholen besteht.

9. Zahnersatzteil nach Anspruch 8, dadurch gekennzeichnet, daß der Kunststoff ein Polymer aus Bis-GMA, äthoxyliertem Bisphenol A-diacrylat, äthoxyliertem Bisphenol A-dimethacrylat, Triäthylenglykoldimethacrylat, Dodecandioldimethacrylat, Urethandimethacrylat aus 2-Hydroxyäthylmethacrylatund 2,2,4-Trimethylhexamethylendiisocyanat, Bis-(acryloyloxymethyl)-tricyclo[$5.2.1.0^{2,6}$]decan und/oder Bis-(methacryloyloxymethyl)-tricyclo[$5.2.1.0^{2,6}$]decan ist.

10. Zahnersatzteil nach Anspruch 8 und 9, dadurch gekennzeichnet, daß der Kunststoff des Kerns ein Polymer aus zu 20 - 85 Gewichts-% aus Bis-GMA oder äthoxyliertem Bisphenol A-dimethacrylat bestehenden Estern der Acrylsäure und/oder Methacrylsäure mit zwei- und/oder mehrwertigen Alkoholen ist.

11. Zahnersatzteil nach Anspruch 8 und 9, dadurch gekennzeichnet, daß der Kunststoff des Mantels ein Polymer aus zu 20 - 70 Gewichts-% aus dem Urethandimethacrylat aus 2-Hydroxyäthylmethacrylat und 2,2,4-Trimethylhexamethylendiisocyanat bestehenden Estern der Acrylsäure und/oder Methacrylsäure mit zwei- und/oder mehrwertigen Alkoholen enthält.

12. Zahnersatzteil nach Anspruch 8 und 9, dadurch gekennzeichnet, daß der Kunststoff des Mantels ein Polymer aus zu 20 - 70 Gewichts-% aus dem Urethandimethacrylat aus 2-Hydroxyäthylmethacrylat und 2,2,4-Trimethylhexamethylendiisocyanat und zu 20 - 40 Gewichts-% aus Bis-(methacryloyloxymethyl)-tricyclo[$5.2.1.0^{2,6}$]decan bestehenden Estern der Acrylsäure und/oder Methacrylsäure mit zwei- und/oder mehrwertigen Alkoholen enthält.

13. Zahnersatzteil nach Anspruch 11 oder 12, dadurch gekennzeichnet, daß der Kunststoff des Mantels zusätzlich ein Polymer aus Dodecandioldimethacrylat enthält.


## Claims

1. A plastics material tooth replacement element, comprising a covering of plastics material consisting of a polymer based on acrylate and/or methacrylate containing 10 to 90 weight percent of highly dispersed silicon dioxide having a particle size of 0.01 - 0.4 micrometres, characterised in that the covering surrounds a core of a plastics material consisting of a polymer based on acrylate and/or methacrylate and containing, as an inorganic filler, 30 - 90 weight percent of a mixture of 60 to 100 weight percent of an inorganic filler material having a mean particle size of 0.7 to 5 micrometres and 0 to 40 weight percent of highly dispersed silicon dioxide having a particle size of 0.01 - 0.4 micrometres.

2. A tooth replacement element according to claim 1, characterised in that the core contains 60 to 85 weight percent of the inorganic filler.

3. A tooth replacement element according to claim 1 or 2, characterised in that the inorganic filler comprises a mixture of 80 - 90 weight percent of inorganic filler material and 10 - 20 weight percent of highly dispersed silicon dioxide.

4. A tooth replacement element according to one of claims 1 to 3, characterised in that the covering contains 30 - 70 weight percent of highly dispersed silicon dioxide.

5. A tooth replacement element according to one of claims 1 to 4, characterised in that the highly dispersed silicon dioxide has a mean particle size of 0.01 to 0.04 micrometres.

6. A tooth replacement element according to one of claims 1 to 5, characterised in that the inorganic filler material consists of silicon dioxide, lithium-aluminium-silicate glass and/or strontium-aluminium silicate glass.

7. A tooth replacement element according to one of claims 1 to 6, characterised in that the inorganic filler material and the highly dispersed silicon dioxide are silanised.

8. A tooth replacement element according to one of claims 1 to 7, characterised in that the plastics material of the core and covering consists of polymeric esters of acrylic acid and/or methacrylic acid with divalent and/or polyvalent alcohols.

9. A tooth replacement element according to claim 8, characterised in that the plastics material is a polymer of Bis-GMA, ethoxylated bisphenol A-diacrylate, ethoxylated bisphenol A-dimethylacrylate, triethyleneglycol dimethacrylate, dodecanediol dimethacrylate, urethane dimethacrylate derived from 2-hydroxyethyl methacrylate and 2,2,4-trimethylhexamethylene di-isocyanate, bis-(acryloyloxymethyl)-tricyclo $[5.2.1.0^{2,6}]$ decane and/or bis (methacryloyloxymethyl)-tricyclo $[5.2.1.0^{2,6}]$ decane.

10. A tooth replacement element according to claims 8 and 9, characterised in that the plastics material of the core is a polymer of esters of acrylic acid and/or methacrylic acid with divalent and/or polyvalent alcohols comprising up to 20 - 85 weight percent of bis-GMA or ethoxylated bisphenol A-dimethacrylate.

11. A tooth replacement element according to claims 8 and 9, characterised in that the plastics material of the covering contains a polymer of esters of acrylic acid and/or methacrylic acid with divalent and/or polyvalent alcohols comprising 20 - 70 weight percent of urethane dimethacrylate derived from 2-hydroxyethyl methacrylate and 2,2,4-trimethylhexamethylene di-isocyanate.

12. A tooth replacement element according to claims 8 and 9, characterised in that the plastics material of the covering contains a polymer of esters of acrylic acid and/or methacrylic acid with divalent and/or polyvalent alcohols comprising 20 - 70 weight percent of urethane dimethacrylate derived from 2-hydroxymethyl methacrylate and 2,2,4-trimethylhexamethylene di-isocyanate and 20 - 40 weight percent of bis(methacryloyloxymethyl)tricyclo$[5.2.1.0^6]$ decane.

13. A tooth replacement element according to claim 11 or 12, characterised in that the plastics material of the covering additionally contains a polymer of dodecanediol dimethacrylate.

## Revendications

1. Prothèse dentaire en matière plastique ayant une enveloppe en matière plastique d'un polymère à base d'acrylate et/ou de méthacrylate, contenant 10-90 % en poids de dioxyde de silicium fortement dispersé d'une dimension de particules de 0,01-0,4 $\mu$ m, caractérisée en ce que l'enveloppe entoure un noyau en matière plastique d'un polymère à base d'acrylate et/ou de méthacrylate contenant comme charge inorganique 30-90 % en poids d'un mélange de 60-100 % en poids d'une matière de charge inorganique d'une dimension moyenne de particules de 0,7-5 $\mu$m et 0-40 % en poids de dioxyde de silicium fortement dispersé d'une dimension de particules de 0,01-0,4 $\mu$m.

2. Prothèse dentaire selon la revendication 1, caractérisée en ce que le noyau contient 60-85 % en poids de la charge inorganique.

3. Prothèse dentaire selon la revendication 1 ou 2, caractérisée en ce que la charge inorganique consiste en un mélange de 80-90 % en poids de matière de charge inorganique et 10-20 % en poids de dioxyde de silicium fortement dispersé.

4. Prothèse dentaire selon l'une des revendications 1 à 3, caractérisée en ce que l'enveloppe contient 30-70 % en poids de dioxyde de silicium fortement dispersé.

5. Prothèse dentaire selon l'une des revendications 1 à 4, caractérisée en ce que le dioxyde de silicium fortement dispersé a une dimension moyenne de particules de 0,01-0,04 μm.

6. Prothèse dentaire selon l'une des revendications 1 à 5, caractérisée en ce que la matière de charge inorganique consiste en dioxyde de silicium, en verre d'aluminosilicate de lithium et/ou en verre d'aluminosilicate de strontium.

7. Prothèse dentaire selon l'une des revendications 1 à 6, caractérisée en ce que la matière de charge inorganique et le dioxyde de silicium fortement dispersé sont silanisés.

8. Prothèse dentaire selon l'une des revendications 1 à 7, caractérisée en ce que la matière plastique du noyau et de l'enveloppe consiste en esters polymères de l'acide acrylique et/ou de l'acide méthacrylique avec des dialcools et/ou des polyalcools.

9. Prothèse dentaire selon la revendication 8, caractérisée en ce que la matière plastique est un polymère de Bis-GMA, de diacrylate de bisphénol A éthoxylé, de diméthacrylate de bisphénol A éthoxylé, de diméthacrylate de triéthylèneglycol, de diméthacrylate de dodécanediol, d'uréthannediméthacrylate du méthacrylate de 2-hydroxyéthyle et du 2,2,4-triméthylhexaméthylènediisocyanate, du bis-(acryloyloxyméthyl)-tricyclo$[5.2.1.0^{2,6}]$décane et/ou du bis-(méthacryloyloxyméthyl)-tricyclo$[5.2.1.0^{2,6}]$-décane.

10. Prothèse dentaire selon les revendications 8 et 9, caractérisée en ce que la matière plastique du noyau est un polymère d'esters d'acide acrylique et/ou d'acide méthacrylique de diol et/ou de polyols consistant pour 20-85 % en poids en Bis-GMA ou en diméthacrylate de bisphénol A éthoxylé.

11. Prothèse dentaire selon les revendications 8 et 9, caractérisée en ce que la matière plastique de l'enveloppe est un polymère d'esters d'acide acrylique et/ou d'acide méthacrylique de diol et/ou de polyol consistant pour 20-70 % en poids en l'uréthannediméthacrylate de méthacrylate de 2-hydroxyéthyle et de 2,2,4-triméthylhexaméthylènediisocyanate.

12. Prothèse dentaire selon les revendications 8 et 9, caractérisée en ce que la matière plastique de l'enveloppe contient un polymère d'esters d'acide acrylique et/ou d'acide méthacrylate de diols et/ou de polyols consistant pour 20-70 % en poids en l'uréthannediméthacrylate de méthacrylate de 2-hydroxyéthyle et de 2,2,4-triméthyhexaméthylènediisocyanate et pour 20-40 % en poids en bis-(méthacryloyloxyméthyl)-tricyclo$[5.2.1.0^{2,6}]$décane.

13. Prothèse dentaire selon la revendication 11 ou 12, caractérisée en ce que la matière plastique de l'enveloppe contient en outre un polymère de diméthacrylate de dodécanediol.

Fig. 1

1

2

Fig. 2

2

1